Europäisches Patentamt

European Patent Office

Office européen des brevets

(19)

(11) **EP 1 042 267 B1**

(12) **EUROPÄISCHE PATENTSCHRIFT**

(45) Veröffentlichungstag und Bekanntmachung des Hinweises auf die Patenterteilung:
**03.09.2003 Patentblatt 2003/36**

(51) Int Cl.$^7$: **C07C 47/04**, C07C 47/058, B01D 1/22

(21) Anmeldenummer: **98954394.7**

(22) Anmeldetag: **12.10.1998**

(86) Internationale Anmeldenummer:
**PCT/EP98/06443**

(87) Internationale Veröffentlichungsnummer:
**WO 99/021818 (06.05.1999 Gazette 1999/18)**

(54) **VERFAHREN UND VORRICHTUNG ZUR HERSTELLUNG VON REINEM FORMALDEHYD**

PROCESS AND DEVICE FOR PREPARING PURE FORMALDEHYDE

PROCEDE ET DISPOSITIF DE PREPARATION DE FORMALDEHYDE PUR

(84) Benannte Vertragsstaaten:
**DE FR GB**

(30) Priorität: **29.10.1997 DE 19747647**

(43) Veröffentlichungstag der Anmeldung:
**11.10.2000 Patentblatt 2000/41**

(73) Patentinhaber: **Ticona GmbH
65451 Kelsterbach (DE)**

(72) Erfinder:
 • **SIEVERS, Werner
 D-65929 Frankfurt am Main (DE)**
 • **SCHWEERS, Elke
 D-69812 Bad Soden (DE)**
 • **ROSENBERG, Michael
 D-65527 Niedernhausen (DE)**

(56) Entgegenhaltungen:
**DE-B- 1 249 846**     **US-A- 2 848 500**
**US-A- 2 943 701**     **US-A- 3 331 191**

 • **DATABASE WPI Section Ch, Week 8719 Derwent Publications Ltd., London, GB; Class J01, AN 87-133278 XP002093027 & JP 62 074402 A (HATTORI K) , 6. April 1987**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

**Beschreibung**

Die vorliegende Erfindung betrifft ein Verfahren sowie eine Vorrichtung zur Nachreinigung insbesondere von Pyrolysegasen zur Herstellung von reinem Formaldehyd.

Zur Herstellung technischer Kunststoffe (Polyacetale, Polyoxymethylen) wird hoch reiner Formaldehyd benötigt. Die Qualität des produzierten, möglichst hochmolekularen Kunststoffes hängt neben den gewählten Polymerisationsbedingungen in erster Linie von der Reinheit des gasförmigen Formaldehyd ab. Es sind verschiedene Verfahren zur Herstellung von Formaldehyd aus Methanol bekannt (z.B. Oxidation im Formox-Prozeß, oxidative Dehydrierung, direkte Dehydrierung von Methanol), die Formaldehyd in flüssiger (wäßriger) oder gasförmiger Phase liefern. Unabhängig vom Phasenzustand (gasförmig oder flüssig) fällt Formaldehyd niemals als reiner Stoff, sondern im Gemisch mit Wasser und anderen Nebenprodukten an. Sowohl für die anionische als auch die kationische Polymerisation wird jedoch gasförmiger Formaldehyd benötigt, der nur minimale Mengen an für die jeweilige Polymerisation schädlichen oder störenden Nebenkomponenten enthalten darf.

Für die Herstellung von hoch reinem, gasförmigem Formaldehyd sind die sogenannten Hemiformalverfahren bekannt. In diesen Verfahren reagiert wäßriger Formaldehyd (ca. 40 - 70 Gew.-% Wasser) mit einem Alkohol unter Bildung von Hemiformalen; z.B. mit Cyclohexanol unter Bildung von Cyclohexyl-Hemiformalen und evtl. Polyformalen. Die Reaktion sieht wie folgt aus:

$$n\ CH_2O + R\text{-}OH \rightarrow R(CH_2O)_nOH$$

Die verwendeten Alkohole sollten keine reaktiven Gruppen wie Carbonyl-, Nitrooder Amin-Gruppen enthalten, die unerwünschte Nebenreaktionen eingehen können. Es können ein- oder mehrwertige Alkohole eingesetzt werden, deren Siedepunkt vorteilhaft deutlich über dem von Wasser liegt. Vorteilhaft ist Cyclohexanol.

Das Hemiformal-Gemisch kann destillativ von Wasser getrennt werden, wobei die über Kopf gehenden Brüden nach Kondensation ein Zweiphasengemisch aus Wasser und Cyclohexanol bilden. Das Sumpfprodukt ist das gewünschte Hemiformal mit geringen Mengen an Wasser, Methanol, Methylformiat und anderen Nebenprodukten.

Das Hemiformal wird nach der Nebenproduktabtrennung thermisch bei 1 bis 1,5 bar und 120 bis 180°C gespalten (Pyrolyse, Thermolyse). Die entstehenden Formaldehyddämpfe werden, wenn noch nicht genügend arm an Nebenprodukten, in einer weiteren Prozeßstufe nachgereinigt. Hier sind zur Abtrennung von Methanol und Wasser sowohl Waschverfahren (Gas strömt entgegen Fallfilm) als auch Prä-Polymerisationsverfahren bekannt. Bei den Waschverfahren wird die Absorptionsfähigkeit von bestimmten Alkoholen ausgenützt (unter Inkaufnahme nennenswerter Formaldehydverluste) und bei den Prä-Polymerisationsverfahren wird ausgenützt, daß bei Polymerisation von Formaldehyd an kalten Oberflächen, wie in der RU 262 45 42 oder der DD 124 92 48 beschrieben, oder kalten Flüssigkeiten, wie in der DE 115 94 20 beschrieben, ein verstärktes Abtrennen von Nebenkomponenten beobachtet werden kann (unter Inkaufnahme der damit einher gehenden Feststoffproblematik). Bei der Prä-Polymerisation werden durch die spontane Bildung niedermolekularen Polymers dem Reaktionsmedium durch die entstehenden Endgruppen Wasser und andere Komponenten entzogen.

Üblicherweise wird die Pyrolyse in einem Verdampfer, z.B. einem Fallfilmverdampfer, durchgeführt. Die Formaldehyddämpfe werden oben am Verdampfer abgezogen. Danach werden die Dämpfe im Gleich- oder Gegenstrom durch Rohre gedrückt, an deren Innenwandung ein Rieselfilm aus beladenem Cyclohexyl-Hemiformal abläuft. Der Rieselfilm ist meist kalt (0 bis 50°C), und absorbiert die störenden Nebenprodukte (z.B. Methanol, Wasser und Methylformiat), aber auch Formaldehyd, was zu quantitativen Verlusten in Höhe von bis zu 30% des eingesetzten Formaldehyds führt. Derartige Verfahren sind beispielsweise in der US 284 85 00 oder der US 294 37 01 beschrieben. Es werden meist nur glatte Rohre verwendet, weil andere Geometrien (z.B. Füllkörper- oder Bodenkolonnen) im Falle ungewollter Polymerisation nicht reinigbar sind. Daneben sind auch andere Absorptionsverfahren bekannt, z.B. mit Polyethylenglykolestern aus der GB 221 8089.

Da bei der Pyrolyse auch Polymerisation beobachtet werden kann, sind erhebliche Anstrengungen zur Bewältigung der Feststoffproblematik unternommen worden. So wurden Pyrolyseapparate in Form von Bodenkolonnen entwickelt, die einzeln beheizte Böden, z.T. mit speziellen, beheizbaren Geometrien, enthalten und beispielsweise in der DD 254 847, der DE 521 33 oder der DD 124 98 46 beschrieben sind. Zur Nachreinigung wurden Verfahren entwickelt, in denen im Fallfilmverdampfer ein beheizter Rotor eingesetzt wird, offenbart in der DD 333 11 91. Um den Prä-Polymerisationseffekt gezielt auszunützen, wurden Tieftemperaturapparaturen (-20°C) entwickelt, in denen zwei beheizbare Schnecken ineinandergreifen und entstehenden Feststoff austragen, bekannt aus der DD 124 92 48.

Um die Nebenprodukte, die in der Cyclohexyl-Hemiformal-Lösung enthalten sind, aus der Flüssigkeit abzutrennen, wurden neben den oben erläuterten Destillationsverfahren auch Adsorptionsverfahren entwickelt, z.B. mit Ionentauscheern zur Adsorption von Ameisensäure und Methylformiat, offenbart in der DD 118 64 53, der IT 119 61 75 oder der IT 311 87 47.

Um die Nebenproduktbildung bei der Pyrolyse, die oft bei hohen Temperaturen von 150 bis 180°C durchgeführt wird, zu vermindern, sind Verfahren bekannt, bei denen Salze in die Cyclohexyl-Hemiformal-Lösung zugegeben werden. Durch die Salzzugabe kann die Bildung hochsiedender Stoffe zurückgedrängt werden, bekannt aus der US 670 50 98 oder der US 670 051 00; andererseits treten Probleme mit Salzverkrustungen auf Wärmetauschern u.a. auf.

Zur Nachreinigung des gasförmigen Formaldehyds ist beispielsweise aus der JP 413 70 54, der DE 305 17 55 oder der IT 318 49 00 auch ein Verfahren bekannt, in dem Wasser adsorptiv aus dem Gasgemisch mit Trockenperlen oder anderen Adsorptionsmitteln (Silicagel, Zeolithe) abgetrennt wird. Auch bei diesem Verfahren ist der Formaldehydverlust sehr hoch, da Formaldehyd auf Adsorptionsmitteln dieser Art adsorbiert wird.

Grundsätzlich entscheidend für eine optimale Pyrolyse sind Temperatur und Druck. Der für die Hemiformalbildung vorgesehene Alkohol sollte deshalb einen genügenden Siedetemperaturabstand zur Pyrolysetemperatur haben.

Alle diese bekannten Verfahren zur Nachreinigung von gasförmigem Formaldehyd weisen jedoch den Nachteil eines erhöhten apparativen Aufwandes infolge einer zum Teil sehr komplizierten konstruktiven Gestaltung mit verschiedenen gekoppelten Vorrichtungen auf, sowie hohe Formaldehydverluste und zum Teil auch unbefriedigend hohe Restverunreinigungen.

Vor diesem Hintergrund ist es daher Aufgabe der vorliegenden Erfindung, ein Verfahren zur Nachreinigung insbesondere von Pyrolysegasen zur Herstellung von reinem Formaldehyd zur Verfügung zu stellen, das die beschriebenen Nachteile überwindet, sowie eine Vorrichtung zur Durchführung des Verfahrens.

Gelöst wird diese Aufgabe erfindungsgemäß durch ein Verfahren zur Nachreinigung insbesondere von Pyrolysegasen zur Herstellung von reinem Formaldehyd, aufweisend die Verfahrensschritte:

- Pyrolysieren von Hemiformal in einem Verdampfer,
- Leiten des entstehenden Formaldehyddampfes durch den im Bodenbereich des Verdampfers gesammelten, kondensierten flüssigen Bestandteil,
- Ableiten des kondensierten flüssigen Bestandteils durch ein gekühltes, im Bodenbereich des Verdampfers angeordnetes Auslaßrohr, so daß der kondensierte flüssige Bestandteil als Fallfilm an der Innenwandung des gekühlten Auslaßrohres abfließt,
- Leiten des Formaldehyddampfes im Gleichstrom durch das gekühlte Auslaßrohr,
- Abziehen des Formaldehyddampfes am vom Verdampfer entfernten Ende des Auslaßrohres, und
- Abziehen des kondensierten flüssigen Bestandteils am vom Verdampfer entfernten Ende des Auslaßrohres.

Obgleich dieses Verfahren mit jedem beliebigen Verdampfer durchgeführt werden kann, wird vorteilhafter Weise in einem Dünnschichtverdampfer pyrolysiert. Die dabei entstehenden Dämpfe werden erfindungsgemäß nicht mehr am oberen Ende des Verdampfers, also über Kopf, abgezogen, sondern in dem als Sumpf bezeichneten Bodenbereich des Verdampfers zusammen mit dem kondensierten flüssigen Bestandteil ausgeschleust. Von diesem im Bodenbereich des Verdampfers angeordneten Sumpf geht ein gekühltes Auslaßrohr ab, in dem zum einen der kondensierte flüssige Bestandteil als Fallfilm an der Innenwandung des Auslaßrohres abfließt, wobei hier beim Pyrolysieren von Hemiformal hauptsächlich aus Alkohol, z.B. Cyclohexanol, besteht. Zum anderen wird der entstandene Formaldehyddampf, vorzugsweise im Gleichstrom, durch das gekühlte Auslaßrohr geleitet und am vom Verdampfer entfernten Ende des Auslaßrohres abgezogen.

Durch den am unteren Ende des Verdampfers in dessen Sumpfbereich ausgeschleusten und im Gleichstrom zum Fallfilm des Alkohols durch das gekühlte Auslaßrohr geleiteten Formaldehyddampf wird sowohl der Fallfilm im Verdampfer als auch der im Auslaßrohr stabilisiert, wobei insbesondere im Verdampfer dabei die Neigung zum Schäumen deutlich verringert wird. Durch eine Gleichstromfahrweise wird darüber hinaus das Mitreißen von Alkoholtröpfchen deutlich verringert, was den Einsatz von Tröpfchenabscheidern oder Kondensatoren im Gegensatz zur Gegenstromfahrweise weitgehend erübrigt.

Die Nebenproduktabtrennung bzw. Nachreinigung findet hier bereits im Sumpf bzw. im Sumpfproduktaustrag des Dünnschichtverdampfers statt. Hier sind dann bei hoher Temperatur Gas und Flüssigkeit in direktem Kontakt, wobei die Temperatur dabei noch nahe der Pyrolysetemperatur ist, die im allgemeinen zwischen 100 und 200°C liegt, vorteilhaft zwischen 145 und 165°C, so daß keine Hemiformale gebildet werden, aber andererseits bereits Nebenkomponenten physikalisch absorbiert werden. Die Übergangszone zwischen dem heißen Verdampfer und dem kalten Auslaßrohr ist extrem kurz, wobei die Temperatur des gekühlten Auslaßrohres im allgemeinen zwischen -20°C und +40°C liegt, vorteilhaft zwischen 0 und 20°C. Das kalte Auslaßrohr dient der Feinreinigung, wobei das Auslaßrohr im allgemeinen im wesentlichen kurz ist, um die Formaldehydverluste klein zu halten. Schließlich sind auch die Formaldehydverluste durch Absorption geringer, da der Verfahrensdruck im Vakuumbereich liegt, vorteilhaft zwischen 600 und 900 mbar, so daß der Partialdruck von Formaldehyd kleiner 1 bar ist.

In einer Weiterbildung ist es auch möglich, den kondensierten flüssigen Bestandteil mit einer Fördervorrichtung in das Auslaßrohr zu fördern.

In einer anderen Weiterbildung ist es auch möglich, einen Teilstrom des kondensierten flüssigen Bestandteils, der arm an Nebenkomponenten (z. B. Trioxan) ist, aus dem Bodenbereich des Verdampfers abzuziehen, während der andere Teilstrom des kondensierten flüssigen Bestandteils durch das gekühlte, im Bodenbereich des Verdampfers angeordnete Auslaßrohr geleitet wird, nach Durchströmen des Auslaßrohres einen höheren Anteil an Nebenkomponenten (z. B. Trioxan) enthält und am vom Verdampfer entfernten Ende des Auslaßrohres abgezogen wird. Auf diese Weise ist es möglich, die beiden flüssigen Teilströme gemäß ihres unterschiedlichen Gehalts an Nebenkomponenten an unterschiedlichen Stellen des Prozesses weiter zu verwenden.

Es ist weiterhin möglich, zwischen der Pyrolysezone und dem Abzug des kondensierten flüssigen Bestandteils, der arm an Nebenkomponenten ist, eine Kühlzone anzubringen, die bei so hohen Temperaturen betrieben wird, daß nur ein Teil des nach Abspaltung von Formaldehyd aus dem Hemiformal entstandenen, verdampften Alkohols, z. B. Cyclohexanol, kondensiert wird. Auf diese Weise lassen sich in vorteilhafter Weise höhere Temperaturen bei der Pyrolyse einstellen, um höhere Abspaltraten an Formaldehyd aus dem Hemiformal zu erzielen und gleichzeitig einen genügend großen Strom an kondensiertem flüssigen Bestandteil. der arm an Nebenkomponenten ist, für die Abnahme zur Verfügung zu haben.

Die Vorrichtung zur Durchführung des Verfahrens zur Nachreinigung insbesondere von Pyrolysegasen zur Herstellung von reinem Formaldehyd weist erfindungsgemäß ein in einem Bodenbereich eines Verdampfers anordbares Auslaßrohr auf, wobei das Auslaßrohr vorzugsweise kühlbar ist und einen Abzug zum Abziehen der im Verdampfer gebildeten Dämpfe aufweist.

Damit wird eine besonders einfache und damit kostengünstige Vorrichtung zur Durchführung des beschriebenen Verfahrens zur Verfügung gestellt, da hier gegenüber den bisher bekannten Verfahren in einer Vorrichtung Alkoholdampf rückkondensiert werden kann. Bei den bisher bekannten und oben ausführlich beschriebenen Verfahren sind hierzu jeweils einzelne Vorrichtungen notwendig, nämlich Verdampfer, Fallfilmrohr und ein nachgeschalteter Kondensator zur Rückkondensation mitgerissenen Alkohols. Weiterhin sind hier Pumpen erforderlich, die Alkohol zur Erzeugung eines Fallfilmes in die Nachreinigungsvorrichtung bzw. das Fallfilmrohr fördern.

Wenngleich das kühlbare Auslaßrohr im Bodenbereich eines beliebigen Verdampfers angeordnet werden kann, so wird es doch vorzugsweise im Bodenbereich eines Dünnschichtverdampfers angeordnet. Wenn dann noch der Verdampfer im Bodenbereich vorteilhafter Weise zum Sammeln des kondensierten, flüssigen Bestandteils ausgebildet ist, kann der erzeugte Formaldehyddampf wie beschrieben verfahrensgemäß durch diesen als Sumpf bezeichneten Bereich zur Nachreinigung hindurch geleitet werden.

Der in diesem Sumpf gesammelte kondensierte, flüssige Bestandteil kann dann in das gekühlte Auslaßrohr gefördert werden, wenn gemäß einer bevorzugten Ausführungsform eine Vorrichtung zum Abfließen des kondensierten, flüssigen Bestandteils im Sumpf angeordnet ist. Auf diese Weise läßt sich besonders einfach der Fallfilm an der Innenwandung des gekühlten Auslaßrohres erzeugen und kontinuierlich aufrechterhalten, wodurch die Nachreinigung wesentlich verbessert wird. Gegebenenfalls können die kondensierten flüssigen Bestandteile auch mit einer Fördervorrichtung gefördert werden. Diese Fördervorrichtung kann beispielsweise als Schneckenförderer ausgebildet sein.

Mit dieser Vorrichtung läßt sich der verfahrensgemäß vorgesehene Gleichstrom des Formaldehyddampfes mit dem Fallfilm im gekühlten Auslaßrohr besonders einfach realisieren, wenn vorteilhafter Weise ein Abzug zum Abziehen der Dämpfe am vom Verdampfer entfernten Ende des Auslaßrohres angeordnet ist. Schließlich ist auch die Kühlung des Auslaßrohres einfach und problemlos möglich, wenn gemäß einer weiteren bevorzugten Ausführungsform das Auslaßrohr doppelwandig ausgebitdet ist.

Der nach dem erfindungsgemäßen Verfahren hergestellte Formaldehyd eignet sich für alle bekannten Einsatzgebiete, beispielsweise Korrosionsschutz, Spiegelherstellung, elektrochemische Beschichtungen, zur Desinfektion und als Konservierungsmittel, ebenso als Zwischenprodukt zur Herstellung von Kunststoffen, beispielsweise Polyoxymethylenen, Polyacetalen, Phenolharzen, Melaminen, Aminoplasten, Polyurethanen und Caseinkunststoffen, 1,4-Butanole, alkoholische Formaldehydlösungen, Methylal, Trimethylolpropan, Neopentylglykol, Pentaerythrit und Trioxan, zur Herstellung von Farbstoffen, wie Fuchsin, Acridin, zur Herstellung von Düngemitteln sowie zur Behandlung von Saatgut.

Nachfolgend wird das Verfahren anhand von Beispielen näher erläutert.

Die in den Beispielen pyrolysierten Hemiformallösungen waren Cyclohexyl-Hemiformale. Hierzu wurde gasförmiger Formaldehyd, der als Rohgas im Gemisch mit Inerten aus einer Methanol-Dehydrierung oder aus einer Trioxanspaltung im Labor hergestellt wurde, in einer Bodenkolonne mit Cyclohexanol zur Hemiformalbildung in Kontakt gebracht. Die Lösungen enthielten ca. 200 - 300 g Formaldehyd (als Hemiformal) pro Liter Lösung. Für die Pyrolyse wurde ein Dünnschichtverdampfer DN50 Rotafilm mit 0,05 m$^2$ Heizfläche eingesetzt. Wenn das Produkt erfindungsgemäß unten abgezogen wurde, befand sich direkt unterhalb des Produktaustrages des Dünnschichtverdampfers ein Rohr mit 250 mm Länge und 25 mm Innendurchmesser, welches über einen Doppelmantel von außen gekühlt werden konnte. Dieses Rohr enthielt den Fallfilm zur Nachreinigung. Wenn das Produkt wie bisher bekannt am oberen Ende des Dünnschichtverdampfers abgezogen wurde, wurden die Formaldehyddämpfe über einen Schlauch in ein Fallfilmrohr geleitet, um nachgereinigt zu werden.

**EP 1 042 267 B1**

in den folgenden Beispielen wurde die Gaszusammensetzung mit einem Gaschromatographen gemessen. Da der Gaschromatograph in reiner Formaldehydatmosphäre keine Nebenprodukte messen kann, wurde der dem Gaschromatographen zugeführte Strom mit Stickstoff verdünnt. Entsprechend sind in den u.a. Tabellen die gemessenen und die um die Verdünnung korrigierten Konzentrationswerte angegeben.

Zunächst wurde das erfindungsgemäße Verfahren mit unten abgezogenem Dampf durchgeführt, wobei der Verdampfer bei verschiedenen Drücken und Temperaturen betrieben wurde. Ebenso wurde die Temperatur des gekühlten Auslaßrohres variiert. Die Ergebnisse sind in der Tabelle 1 zusammengefaßt.

Tab. 1:

| Versuch: FAR01 | | | | | | | |
|---|---|---|---|---|---|---|---|
| Produkt unten abgezogen | | | | | | | |
| Dünnschichtverdampfer DN50 Rotafilm mit 0,05m$^2$ | | | | | | | |
| Fallfilm: Rohrlänge 250 mm | | | | | | | |
| Feed: | Vol.-Strom: | 0,2 l/h | | | | | |
| | Formaldehyd: | 240 g/l | | | | | |
| Drehzahl: | 900 1/min | | | | | | |
| Druck Verdampfer [mbar] | Temperatur Verdampfer [°C] | Temperatur Fallfilm [°C] | Formaldehyd-Dampf Vol-strom [l/h] | Formaldehyd [%] | Wasser [ppm] | Methanol [ppm] | Methylformiat [ppm] |
| 800 | 150 | 20 | 20 | 40 | 2000 | 20 | 1000 |
| 600 | 145 | 0 | 20 | 40 | 1800 | 15 | 1000 |
| 600 | 170 | -9 | 17 | 50 | 1500 | 12 | 2200 |
| | | | | | | | |
| **korrigiert auf 100% Formaldehyd, da ursprünglich mit Stickstoff verdünnt:** | | | | | | | |
| Druck Verdampfer [mbar] | Temperatur Verdampfer [°C] | | Formaldehyd-Dampf Vol-strom [l/h] | Formaldehyd [%] | Wasser [ppm] | Methanol [ppm] | Methylformiat [ppm] |
| 800 | 150 | 20 | 20 | 100 | 5000 | 50 | 2500 |
| 600 | 145 | 0 | 20 | 100 | 4500 | 38 | 2500 |
| 600 | 170 | -9 | 17 | 100 | 3000 | 24 | 4400 |

Es konnten im Produktgas Methanol-Konzentrationen von nur 24 ppm erreicht werden. Gut zu erkennen ist, daß mit fallender Temperatur im Fallfilm besser nachgereinigt wird. Die Nebenproduktbildung im Dünnschichtverdampfer steigt zwar durch den Temperatursprung von 145 auf 170°C an, was aber bei Methanol gut durch das beschriebene Verfahren ausgeglichen wird. Weiterhin kann gezeigt werden, daß der Formaldehydverlust zwischen 20 und 0°C im Fallfilm unerheblich ist (jeweils 20 l/h Produkt). Der Fallfilm sollte also vorteilhaft bei 0 bis 10°C betrieben werden.

Im Beispiel 2 wurde der Dampf entsprechend dem Stand der Technik am Kopf des Dünnschichtverdampfers abgezogen und nicht nachgereinigt. Die Ergebnisse dieses Beispiels sind in der Tabelle 2 aufgeführt.

Tab.2:

| Versuch: PYR9 | | | | | |
|---|---|---|---|---|---|
| Produkt oben abgezogen | | | | | |
| Dünnschichtverdampfer DN50 Rotafilm mit 0,05m$^2$ | | | | | |
| Feed: | Vol.-Strom: | 0,4 l/h | | | |
| | Formaldehyd: | 160 g/l | | | |
| Drehzahl: | 900 1/min | | | | |
| Druck Verdampfer [mbar] | Temperatur Verdampfer [°C] | Formaldehyd-DampfVol-strom [l/h] | Formaldehyd [%] | Methanol [ppm] | Methylformiat [ppm] |
| 800 | 154 | 7,5 | 701 | 350 | 1000 |
| 600 | 144 | 7,5 | 70 | 300 | 1000 |
| 400 | 125 | 4 | 20 | 250 | 300 |
| 100 | 125 | 1 | 8 | 300 | 90 |
| | | | | | |
| **korrigiert auf 100% Forma dehyd, da ursprünglich mit Stickstoff verdünnt:** | | | | | |
| Druck Verdampfer [mbar] | Temperatur Verdampfer [°C] | Formaldehyd-DampfVol-strom [l/h] | Formaldehyd [%] | Methanol [ppm] | Methylformiat [ppm] |
| 800 | 154 | 7,5 | 100 | 500 | 1429 |
| 600 | 144 | 7,5 | 100 | 429 | 1429 |
| 400 | 125 | 4 | 100 | 1250 | 1500 |
| 100 | 125 | 1 | 100 | 3750 | 1125 |

Die Methanol-Konzentrationen sind im Bereich von mehreren 100 ppm. Aus diesem Beispiel kann eine optimierte Verfahrensweise für die Pyrolyse gezeigt werden. Bei einem Druck von 600 bis 800 mbar und einer Temperatur von 145 bis 160°C kann das Cyclohexyl-Hemiformal mit maximaler Ausbeute (siehe Formaldehyd-Volumenströme in Tab.2) und niedrigen Nebenproduktanteilen pyrolysiert werden.

In Beispiel 3 wurde das Gas am Kopf des Dünnschichtverdampfers abgezogen und über einen Schlauch in ein 250 mm langes Rohrstück geleitet, welches zuerst auf 20°C und dann auf 0°C gekühlt wurde. Es bildet sich eine schwacher Rieselfilm aus kondensiertem Cyclohexanol aus, der seinerseits einen Nachreinigungseffekt aufweist, siehe Tab.3.

Tab.3:

| Versuch: PYR13 | | | | | | | | |
|---|---|---|---|---|---|---|---|---|
| Produkt oben abgezogen | | | | | | | | |
| Dünnschichtverdampter DN50 Rotafilm mit 0,05m$^2$ | | | | | | | | |
| Fallfilm: Rohrlänge 250 mm | | | | | | | | |
| Feed: | Vol.-Strom: | 0,2 l/h | | | | | | |
| | Formaldehyd: | 270 g/l | | | | | | |
| Urehzahl: | 900 1/mm | | | | | | | |
| Druck Verdampfer [mbar] | Temperatur Verdampfer [°C] | Temperatur Fallfilm [°C] | Formaldehyd-Dampf Vol.-strom [l/h] | Formaldehyd [%] | Wasser [ppm] | Methanol [ppm] | Methylformiat [ppm] | |
| 800 | 145 | 20 | 11 | 35 | 1500 | 45 | 400 | |
| 800 | 145 | 0 | 11 | 35 | 1500 | 45 | 400 | |
| | | | | | | | | |
| **korrigiert auf 100% Formaldehyd, da ursprunglich mit Stickstott verdünnt:** | | | | | | | | |
| Druck Verdampfer [mbar] | Temperatur Verdampfer [°C] | | Formaldehyd-Dampf Vol.-strom [l/h] | Formaldehyd [%] | Wasser [ppm] | Methanol [ppm] | Methylformiat [ppm] | |
| 800 | 145 | 20 | 11 | 100 | 4286 | 129 | 1143 | |
| 800 | 145 | 0 | 11 | 100 | 4286 | 129 | 1143 | |

Im Beispiel 4 wurde das Produkt zwar erfindungsgemäß unten abgezogen, aber dann nicht in ein direkt an den Dünnschichtverdampfer angeflanschtes Fallfilmrohr geleitet, sondern über einen Schlauch in ein separates Rohr geführt. Das 1 m lange und 25 mm im Durchmesser messende Rohr kann von außen gekühlt werden. Die Nachreinigung in diesem als Fallfilmapparat eingesetzten Rohr wurde gemäß der US 294 37 01 betrieben. Hierzu wurde ein beladenes Cyclohexyl-Hemiformal-Gemisch bei 0°C auf den Kopf des Rohres gepumpt, um als Rieselfilm an der Innenwandung des Rohres abzulaufen. Das Gas aus der Pyrolyse wurde im Gegenstrom dazu von unten nach oben durch das Rohr gedrückt. Gemessen wurden die Gaskonzentrationen jeweils direkt nach der Pyrolyse und nach der Nachreinigung, siehe Tab.4.

Tab.4:

| Versuch: PYR12 | | | | |
|---|---|---|---|---|
| Produkt unten abgezogen | | | | |
| Dünnschichtverdampfer DN50 Rotafilm mit 0.05m$^2$ | | | | |
| extern autgebauter Fallfilm: Kohrlänge 1000 mm, DN25, gemäß Patent H25 | | | | |
| Feed: | Vol.-Strom: | 0,2 l/h | | |
| | Formaldehyd: | 282 g/l | | |
| Drehzahl: | 900 1/min | | | |
| Druck Verdampfer | 800 mbar | | | |
| Temperatur Verdampfer | 155 °C | | | |
| Temperatur Fallfilm | 0°C | | | |
| | Formaldehyd-Dampf Vol.-strom [l/h] | Formaldehyd [%] | Methanol [ppm] | Methylformiat [ppm] |
| vor Fallfilm | 45 | 100 | 150 | 4500 |
| nach Fallfilm | 8 | 100 | 360 | 2000 |

Die Formaldehydverluste waren enorm hoch. Es wurden ca. 80% des produzierten Formaldehyd zu Hemiformalen umgesetzt. Da der Gasstrom deswegen von 45 l/h auf 8 l/h abnahm, stieg sogar der Methanolgehalt von 150 auf 360 ppm.

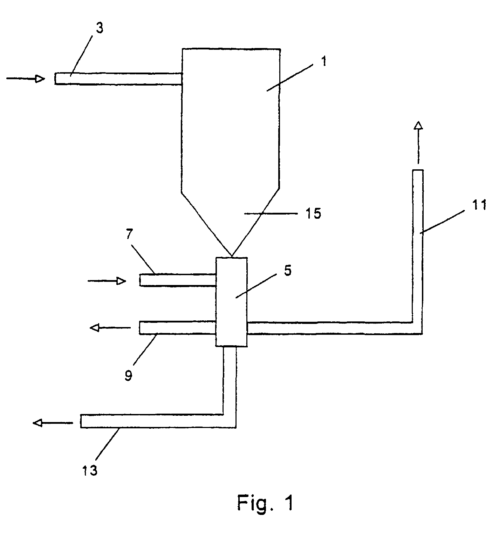

Eine Vorrichtung zur Durchführung des Verfahrens wird anhand der Figur 1 erläutert, die eine schematische Darstellung zeigt: Abgebildet ist ein Dünnschichtverdampfer 1, der am oberen Ende eine Hemiformalzuleitung 3 aufweist. Der untere Bereich des Dünnschichtverdampfers 1 ist als Sumpf 15 ausgebildet. Der Sumpf 15 mündet in ein Auslaßrohr 5, welches hier doppelwandig ausgebildet ist und eine Kühlmittelzuleitung 7 sowie eine Kühlmittelableitung 9 aufweist, durch die das Kühlmittel zum Kühlen heran- bzw. abgeführt wird. Am unteren Ende des Auslaßrohres 5 ist weiterhin ein Abzug 11 zum Ableiten des hoch reinen Formaldehydgases angeordnet. Schließlich weist das Auslaßrohr 5 am unteren Ende noch einen Abzug 13 des kondensierten flüssigen Bestandteils auf Zusätzlich ist ein Abzug 17 für einen Teilstrom des kondensierten flüssigen Bestandteils aus dem Sumpf 15 des Dünnschichtverdampfers 1 angebracht.

Das Hemiformal wird über die Zuleitung 3 dem Dünnschichtverdampfer 1 zugeleitet und pyrolysiert. Dabei sammeln sich im Sumpf 15 die kondensierten flüssigen Bestandteile, hier insbesondere Alkohol, z.B. Cyclohexanol. Dieses wird vom Sumpf 15 in das Auslaßrohr 5 geleitet, wobei es an dessen Innenwandung als Fallfilm abfließt. Ein Teilstrom der kondensierten flüssigen Bestandteile kann über den Abzug 17 entnommen und einem anderen Prozeßschritt zugeführt werden. Die im Verdampfer gebildeten Formaldehyddämpfe werden ebenfalls durch den Sumpf 15 in das Auslaßrohr 5 geleitet und dort im Gleichstrom zum Fallfilm des Alkohols bis zum Abzug 11 geführt. Dort werden sie abgezogen und als hoch reines Formaldehydgas zur Polymerisation weitergeleitet. Der als Fallfilm im Auslaßrohr 5 abgeleitete Alkohol wird durch den Abzug 13 entnommen und einem anderen Prozeßschritt zugeführt. Die Kühlung des Auslaßrohres 5 erfolgt dabei über einen Kühlkreislauf, an den das Auslaßrohr 5 mittels der Kühfmittelzuleitung 7 bzw. der Kühlmittelableitung 9 angeschlossen ist.

Mit dieser beschriebenen Vorrichtung ist es möglich, Pyrolyse und Nachreinigung des erzeugten Formaldehyddampfes in einer Vorrichtung durchzuführen. Damit wird der apparative Aufwand deutlich reduziert, so daß insgesamt die Kosten deutlich reduziert werden. Weiterhin entstehen bei Verwendung dieser Vorrichtung und Anwendung des beschriebenen Verfahrens nur geringe Formaldehydverluste, und es wird eine hohe Gasreinheit erzielt. So wird beispielsweise die Herstellung von gasförmigem Formaldehyd mit Methanolanteilen kleiner 40 ppm möglich.

Bezugszeichenliste

1    Dünnschichtverdampfer
3    Hemiformalzuleitung
5    Auslaßrohr
7    Kühlmittelzuleitung
9    Kühlmittelableitung
11   Abzug des hochreinen Formaldehydgases
13   Abzug des kondensierten flüssigen Bestandteils nach dem Auslaßrohr
15   Sumpf
17   Abzug des kondensierten flüssigen Bestandteils aus dem Sumpf des Verdampfers


**Patentansprüche**

1.  Verfahren zur Nachreinigung insbesondere von Pyrolysegasen zur Herstellung von reinem Formaldehyd, aufweisend die Verfahrensschritte:

    -   Pyrolysieren von Hemiformal in einem Verdampfer,
    -   Leiten des entstehenden Formaldehyddampfes durch den im Bodenbereich des Verdampfers gesammelten, kondensierten flüssigen Bestandteil,
    -   Ableiten des kondensierten flüssigen Bestandteils durch ein gekühltes, im Bodenbereich des Verdampfers angeordnetes Auslaßrohr, so daß der kondensierte flüssige Bestandteil als Fallfilm an der Innenwandung des gekühlten Auslaßrohres abfließt,
    -   Leiten des Formaldehyddampfes im Gleichstrom durch das gekühlte Auslaßrohr,
    -   Abziehen des Formaldehyddampfes am vom Verdampfer entfernten Ende des Auslaßrohres und
    -   Abziehen des kondensierten flüssigen Bestandteils am vom Verdampfer entfernten Ende des Auslaßrohres.

2.  Verfahren zur Nachreinigung von Nebenkomponenten enthaltenden Pyrolysegasen zur Herstellung von reinem Formaldehyd, aufweisend die Verfahrensschritte:

    -   Pyrolysieren von Hemiformal in einem Verdampfer,
    -   Leiten des entstehenden Formaldehyddampfes durch den im Bodenbereich des Verdampfers gesammelten, kondensierten flüssigen Bestandteil, der arm an Nebenkomponenten ist,
    -   Gegebenenfalls Vorkühlen des Dampfes, so daß ein Teil des nach Abspaltung von Formaldehyd aus dem Hemiformal vorliegenden, verdampften Alkohols kondensiert wird,
    -   Abziehen eines Teils des kondensierten flüssigen Bestandteils, der arm an Nebenkomponenten ist, aus dem Bodenbereich des Verdampfers,
    -   Ableiten des anderen Teils des kondensierten flüssigen Bestandteils durch ein gekühltes, im Bodenbereich des Verdampfers angeordnetes Auslaßrohr, so daß dieser Teil des kondensierten flüssigen Bestandteils als Fallfilm an der Innenwandung des gekühlten Auslaßrohres abfließt,
    -   Leiten des Formaldehyddampfes im Gleichstrom durch das gekühlte Auslaßrohr,
    -   Abziehen des Formaldehyddampfes am vom Verdampfer entfernten Ende des Auslaßrohres, und
    -   Abziehen des anderen Teils des kondensierten flüssigen Bestandteils, der nach Durchströmen des Auslaßrohres einen höheren Anteil an Nebenkomponenten enthält, am vom Verdampfer entfernten Ende des Auslaßrohres.

3.  Verfahren nach Anspruch 1 oder 2, **dadurch gekennzeichnet, daß** der kondensierte, flüssige Bestandteil von selbst (durch Schwerkraft) in das Auslaßrohr abläuft.

4.  Verfahren nach Anspruch 1 oder 2, **dadurch gekennzeichnet, daß** der kondensierte flüssige Bestandteil mit einer Fördervorrichtung in das Auslaßrohr gefördert wird.

5.  Verfahren nach einem der vorangehenden Ansprüche, **dadurch gekennzeichnet, daß** die Pyrolysetemperatur zwischen 100 und 200°C liegt.

**6.** Verfahren nach einem der vorangehenden Ansprüche, **dadurch gekennzeichnet, daß** die Temperatur des gekühlten Auslaßrohres zwischen -20°C und +40°C liegt.

**7.** Verfahren nach einem der vorangehenden Ansprüche, **dadurch gekennzeichnet, daß** der Verfahrensdruck im Vakuumbereich liegt.

**8.** Vorrichtung zur Nachreinigung insbesondere von Pyrolysegasen zur Herstellung von reinem Formaldehyd mit einem im Bodenbereich eines Verdampfers (1) anordbaren Auslaßrohr (5), wobei das Auslaßrohr (5) kühlbar ist und einen Abzug (11) zum Abziehen der im Verdampfer (1) gebildeten Oämpfe aufweist.

**9.** Vorrichtung nach Anspruch 8, **dadurch gekennzeichnet, daß** der Verdampfer (1) ein Dünnschichtverdampfer ist.

**10.** Vorrichtung nach Anspruch 8 oder 9, **dadurch gekennzeichnet, daß** der Verdampfer (1) im Bodenbereich (15) zum Sammeln des kondensierten flüssigen Bestandteils ausgebildet ist.

**11.** Vorrichtung nach Anspruch 10, **dadurch gekennzeichnet, daß** im Sammelbereich (15) des Verdampfers (1) eine Vorrichtung zum Fördern des kondensierten flüssigen Bestandteils in das Auslaßrohr (5) angeordnet ist.

**12.** Vorrichtung nach Anspruch 11, **dadurch gekennzeichnet, daß** die Fördervorrichtung ein Schneckenförderer ist.

**13.** Vorrichtung nach einem der Ansprüche 8 bis 12, **dadurch gekennzeichnet, daß** der Abzug (11) zum Abziehen der Dämpfe am vom Verdampfer (1) entfernten Ende des Auslaßrohres (5) angeordnet ist.

**14.** Vorrichtung nach einem der Ansprüche 8 bis 13, **dadurch gekennzeichnet, daß** das Auslaßrohr (5) doppelwandig ausgebildet ist.

**Claims**

**1.** A process for the post-purification in particular of pyrolysis gases for preparing pure formaldehyde, having the process steps of:

- pyrolyzing hemiformal in an evaporator,
- passing the resulting formaldehyde vapor through the condensed liquid component collected in the bottom area of the evaporator,
- passing out the condensed liquid component through a cooled outlet tube disposed in the bottom area of the evaporator, so that the condensed liquid component flows down as a falling film on the inner wall of the cooled outlet tube,
- passing the formaldehyde vapor in cocurrent through the cooled outlet tube,
- taking off the formaldehyde vapor at the outlet tube end remote from the evaporator, and
- taking off the condensed liquid component at the outlet tube end remote from the evaporator.

**2.** A process for the post-purification of pyrolysis gases comprising minor components for preparing pure formaldehyde, having the process steps of:

- pyrolyzing of hemiformal in an evaporator,
- passing the resulting formaldehyde vapor through the condensed liquid component collected in the bottom area of the evaporator, which is low in minor components,
- if appropriate precooling the vapor, so that some of the vaporized alcohol present after elimination of formaldehyde from the hemiformal is condensed,
- taking off some of the condensed liquid component which is low in minor components from the bottom area of the evaporator,
- passing out the other portion of the condensed liquid component through a cooled outlet tube disposed in the bottom area of the evaporator, so that this portion of the condensed liquid component flows down as a falling film on the inner wall of the cooled outlet tube,
- passing the formaldehyde vapor in cocurrent through the cooled outlet tube,
- taking off the formaldehyde vapor at the outlet tube end remote from the evaporator, and
- taking off the other portion of the condensed liquid component, which after flowing through the outlet tube has a higher content of minor components, at the outlet tube end remote from the evaporator

3. The process as claimed in claim 1 or 2, wherein the condensed liquid component runs out spontaneously (by gravity) into the outlet tube.

4. The process as claimed in claim 1 or 2, wherein the condensed liquid component is transported into the outlet tube using a transport apparatus.

5. The process as claimed in one of the preceding claims, wherein the pyrolysis temperature is between 100 and 200°C.

6. The process as claimed in one of the preceding claims, wherein the temperature of the cooled outlet tube is between -20°C and +40°C.

7. The process as claimed in one of the preceding claims, wherein the process pressure is in the vacuum range.

8. An apparatus for the post-purification in particular of pyrolysis gases for preparing pure formaldehyde having an outlet tube (5) which is disposable in the bottom area of an evaporator (1), the outlet tube (5) being coolable and having a takeoff (11) for taking off the vapors formed in the evaporator (1).

9. The apparatus as claimed in claim 8, wherein the evaporator (1) is a thin-film evaporator.

10. The apparatus as claimed in claim 8 or 9, wherein the evaporator (1) is constructed in the bottom area (15) for collecting the condensed liquid component.

11. The apparatus as claimed in claim 10, wherein an apparatus for transporting the condensed liquid component into the outlet tube (5) is disposed in the collection area (15) of the evaporator (1).

12. The apparatus as claimed in claim 11, wherein the transport apparatus is a screw conveyor.

13. The apparatus as claimed in one of claims 8 to 12, wherein the takeoff (11) for taking off the vapors is disposed at the outlet tube (5) end remote from the evaporator (1).

14. The apparatus as claimed in one of claims 8 to 13, wherein the outlet tube (5) is of double-wall construction.

**Revendications**

1. Procédé d'épuration, en particulier de gaz de pyrolyse pour la préparation de formaldéhyde pur, qui présente les étapes de procédé suivantes:

   - pyrolyse d'hémiformal dans un évaporateur,
   - passage de la vapeur de formaldéhyde résultante à travers le composant liquide condensé rassemblé dans la région du fond de l'évaporateur,
   - évacuation du composant liquide condensé par un tube de sortie refroidi disposé dans la région du fond de l'évaporateur de telle sorte que le composant liquide condensé s'écoule en film sur la paroi intérieure du tube de sortie refroidi,
   - passage de la vapeur de formaldéhyde en courant parallèle dans le tube de sortie refroidi,
   - extraction de la vapeur de formaldéhyde à l'extrémité du tube de sortie éloignée de l'évaporateur et
   - extraction du composant liquide condensé à l'extrémité du tube de sortie éloignée de l'évaporateur.

2. Procédé d'épuration de gaz de pyrolyse contenant des composants secondaires, pour la préparation de formaldéhyde pur, qui présente les étapes de procédé suivantes:

   - pyrolyse d'hémiformal dans un évaporateur,
   - passage de la vapeur de formaldéhyde résultante à travers le composant liquide condensé rassemblé dans la région du fond de l'évaporateur, et pauvre en composants secondaires,
   - éventuellement, pré-refroidissement de la vapeur de telle sorte qu'une partie de l'alcool qui s'est évaporé après la dissociation du formaldéhyde à partir de l'hémiformal se condense,

- extraction d'une partie du composant liquide condensé pauvre en composants secondaires hors de la région du fond de l'évaporateur,

- évacuation de l'autre partie du composant liquide condensé par un tube de sortie refroidi disposé dans la région du fond de l'évaporateur de telle sorte que cette partie du composant liquide condensé s'écoule en film sur la paroi intérieure du tube de sortie refroidi,

- passage de la vapeur de formaldéhyde en courant parallèle dans le tube de sortie refroidi,

- extraction de la vapeur de formaldéhyde à l'extrémité du tube de sortie éloignée de l'évaporateur et

- extraction de l'autre partie du composant liquide condensé, qui après avoir traversé le tube de sortie contient une proportion plus élevée en composants secondaires, à l'extrémité du tube de sortie éloignée de l'évaporateur.

3. Procédé selon la revendication 1 ou 2, **caractérisé en ce que** le composant liquide condensé s'écoule de lui-même (par gravité) dans le tube de sortie.

4. Procédé selon la revendication 1 ou 2, **caractérisé en ce que** le composant liquide condensé est transporté dans le tube de sortie au moyen d'un dispositif de transport.

5. Procédé selon l'une des revendications précédentes, **caractérisé en ce que** la température de pyrolyse est comprise entre 100 et 200°C.

6. Procédé selon l'une des revendications précédentes, **caractérisé en ce que** la température du tube de sortie refroidi est comprise entre -20°C et +40°C.

7. Procédé selon l'une des revendications précédentes, **caractérisé en ce que** la pression de procédé est située dans la plage du vide.

8. Dispositif d'épuration, en particulier de gaz de pyrolyse pour la préparation de formaldéhyde pur, avec un tube de sortie (5) apte à être agencé dans la région du fond d'un évaporateur (1), le tube de sortie (5) pouvant être refroidi et présentant une extraction (11) pour extraire les vapeurs formées dans l'évaporateur (1).

9. Dispositif selon la revendication 8, **caractérisé en ce que** l'évaporateur (1) est un évaporateur à couche mince.

10. Dispositif selon la revendication 8 ou 9, **caractérisé en ce que** la région (15) du fond de l'évaporateur (1) est configurée de manière à rassembler le composant liquide condensé.

11. Dispositif selon la revendication 10, **caractérisé en ce que** dans la région de collecte (15) de l'évaporateur (1) est disposé un dispositif pour le transport du composant liquide condensé dans le tube de sortie (5).

12. Dispositif selon la revendication 11, **caractérisé en ce que** le dispositif de transport est un transporteur à vis.

13. Dispositif selon l'une des revendications 8 à 12, **caractérisé en ce que** l'extraction (11) pour extraire les vapeurs est disposée à l'extrémité du tube de sortie (5) éloignée de l'évaporateur (1).

14. Dispositif selon l'une des revendications 8 à 13, **caractérisé en ce que** le tube de sortie (5) est réalisé à double paroi.

Fig. 1